# EUROPEAN PATENT APPLICATION

(11) **EP 3 701 858 A1**
(43) Date of publication of application: **02.09.2020**
(21) Application number: 20169974.1
(22) Date of filing: 01.07.2014
(51) Int. Cl.: A61B 5/0205, A61B 5/0408, A61B 5/04, A61B 5/00, A61B 5/0416, A61B 5/1171, A61B 5/0478, A61B 5/0492, A61B 5/053, A61B 5/117, A61B 5/145, A61B 5/1455

(54) **ADVANCED HEALTH MONITORING SYSTEM**

(30) Priority: 01.07.2013 US 201361841837 P; 01.07.2013 US 201361841865 P
(62) Divisional of application: 14820654.3
(71) Applicant: Mayo Foundation for Medical Education and Research, Rochester, MN 55905 (US)
(72) Inventor: Bennet, Kevin E., Rochester, MN 55902 (US)
(74) Representative: Väänänen, Mikko Kalervo

(57) **Abstract**

Devices and methods provided herein can enhance the operations and efficacy of a remote patient monitoring system. Such devices and methods include using a wearable monitoring device that may comprise non-invasive sensors as well as microsensors that are positioned subdermally in some embodiments. In some embodiments, the wearable health parameter monitoring devices include a disposable component and a reusable component that are electrically and mechanically coupled together to create a functioning monitor device.

## Description

### CROSS-REFERENCE TO RELATED APPLICATIONS

This application claims the benefit of U.S. Provisional Application Serial No. 61/841,837, filed July 1, 2013, and U.S. Provisional Application Serial No. 61/841,865, filed July 1, 2013. The disclosures of those prior applications are considered part of (and are incorporated by reference in) the disclosure of this application.

### BACKGROUND

### 1. Technical Field

This document relates to devices and methods for the remote monitoring of patient health parameters. For example, this document relates to the use of devices, methods, and algorithms to enhance the operations and efficacy of a remote patient monitoring system. This document also relates to devices and methods for monitoring health parameters using a wearable monitoring device that may include non-invasive sensors as well as microsensors that are positioned subdermally in some embodiments.

### 2. Background Information

For a variety of reasons, the importance of remote health monitoring systems, such as in-home monitoring systems, is increasing. Remote health parameter monitoring of ambulatory patients enables doctors to detect or diagnose health problems, such as heart arrhythmias, that may produce only transient symptoms and, therefore, may not be evident when the patients visit the doctors' offices. Remote health parameter monitoring is a significant tool available to healthcare providers for reducing hospital readmission rates and to track disease progression. The use of monitoring systems can permit a smooth transition from hospital to home care. Steadily increasing healthcare costs and outpatient populations have created a need to maximize time intervals between office visits.

The relentless pressure to reduce costs in the healthcare industry has required the more efficient use of a healthcare professional's services. As a result, many physicians now regularly prescribe home monitoring of such health parameters as blood pressure, heart rate, blood glucose level, and EKG (electrocardiogram) signals. In addition, health insurance providers are increasingly viewing remote health parameter monitoring as a means to reduce in-patient expenses and overall healthcare costs.

### SUMMARY

This document provides devices and methods for the remote monitoring of patient health parameters. For example, this document provides devices, methods, and algorithms to enhance the operations and efficacy of a remote patient monitoring system. This document also provides devices and methods for monitoring health parameters using a wearable monitoring device that may include non-invasive sensors as well as microsensors that are positioned subdermally in some embodiments.

In general, one aspect of this document features a device for monitoring health parameters of a patient. The device comprises: a sensor patch in contact with a skin surface of the patient, wherein the sensor patch comprises a plurality of sensors for measuring physiologic or pathologic parameters of the patient; a control unit, wherein the control unit is releasably receivable in a cradle of the sensor patch, and wherein the control unit is in electrical communication with the plurality of sensors when the control unit is in the cradle; and a cap, wherein the cap is configured to releasably couple with the sensor patch to detain the control unit in the cradle, and wherein the cap includes a user interface that is configured to provide indications of the functioning of the device.

In various implementations, at least one of the plurality of sensors may be configured to penetrate the skin and be at least partially disposed below the surface of the skin. The device may further comprise a reservoir that is configured to contain a liquid drug, wherein the device is configured to dispense an amount of the liquid drug to the patient below the surface of the skin. The device may further comprise one or more accelerometers, wherein the accelerometers are configured to provide signals used to determine physical motion of the patient. At least one accelerometer may be located below the surface of the skin.

In another general aspect, this document features a system for monitoring health parameters of a patient, the system comprising: a sensor patch in contact with a skin surface of the patient, wherein the sensor patch comprises a plurality of sensors for measuring physiologic or pathologic parameters of the patient; a control unit, wherein the control unit is releasably receivable in a cradle of the sensor patch, and wherein the control unit is in electrical communication with the plurality of sensors when the control unit is in the cradle; a cap, wherein the cap is configured to mechanically couple with the sensor patch to detain the control unit in the cradle, and wherein the cap includes a user interface that is configured to provide indications of the functioning of the device; and a computing device, wherein the computing device is configured to electrically communicate with the control unit when the control unit is not in the cradle and to receive data from the control unit, and wherein the computing device is configured to transmit the data or derivatives of the data to another computing device over a network.

In various implementations, the computing device may be a personal computer. The computing device may be a cellular telephone. The network may be the internet.

In another general aspect, this document features a method of determining and using a preferred anatomical site at which to locate sensors of a health parameter monitoring system on a patient. The method comprises: recording health parameter data of the patient from a first anatomical site on the patient to create a first data set; recording health parameter data of the patient from a second anatomical site on the patient to create a second data set, wherein the first and second anatomical sites are different locations on the patient; comparing the first data set with the second data set; determining which of the first data set or the second data set represents a preferred signal; selecting the first anatomical site as the preferred anatomical site based on determining that the first data set represents the preferred signal or selecting the second anatomical site as the preferred anatomical site based on the determining that the second data set represents the preferred signal; recording, using the health parameter monitoring system, health parameter data of the patient from an anatomical site that is established by the patient's application of the monitoring system to the patient's anatomy to create a third data set; comparing the third data set to the data set from the preferred anatomical site; and based on the comparison of the third data set to the data set from the preferred anatomical site, determining whether the anatomical site that is established by the patient's application of the monitoring system to the patient's anatomy is sufficiently similar to the preferred anatomical site.

In various implementations, the health parameter monitoring system may comprise: a sensor patch that is configured for being in contact with a skin surface of the patient, wherein the sensor patch comprises a plurality of sensors for measuring physiologic or pathologic parameters of the patient; a control unit, wherein the control unit is releasably receivable in a cradle of the sensor patch, and wherein the control unit is in electrical communication with the plurality of sensors when the control unit is in the cradle; and a cap, wherein the cap is configured to releasably couple with the sensor patch to detain the control unit in the cradle, and wherein the cap includes a user interface that is configured to provide indications related to the functioning of the health parameter monitoring system.

In another general aspect, this document features a health parameter monitoring system comprising: a sensor patch configured to be in contact with a human skin surface, wherein the sensor patch comprises a plurality of sensors for measuring physiologic or pathologic parameters; a control unit that is releasably receivable in a cradle of the sensor patch, wherein the control unit is in electrical communication with the plurality of sensors when the control unit is in the cradle, wherein the control unit comprises a data processing device that is encoded with a computer program, the computer program comprising instructions that when executed cause (i) data measured by the plurality of sensors to be compared to triggering events that comprise threshold values for health parameters and (ii) an initiation of one or more countermeasures in response to a determination that the data measured by the plurality of sensors meets or exceeds the threshold values of the triggering events; and a cap, wherein the cap is configured to releasably couple with the sensor patch to detain the control unit in the cradle, and wherein the cap includes a user interface that is configured to provide indications related to the functioning of the health parameter monitoring system.

In various implementations, the countermeasures may include alerting the patient. The countermeasures may include alerting a remote monitoring service in communication with the health parameter monitoring system.

In another general aspect, this document features a method of operating a health parameter monitoring system. The method comprises: providing the health parameter monitoring system and applying the sensor patch to a skin surface of a patient, wherein the sensor patch administers the pharmacological agent to the patient. The health parameter monitoring system comprises: a sensor patch configured to be in contact with a human skin surface, wherein the sensor patch comprises a plurality of sensors for measuring physiologic or pathologic parameters and a pharmacological agent; a control unit, wherein the control unit is releasably receivable in a cradle of the sensor patch, and wherein the control unit is in electrical communication with the plurality of sensors when the control unit is in the cradle; and a cap, wherein the cap is configured to releasably couple with the sensor patch to detain the control unit in the cradle, and wherein the cap includes a user interface that is configured to provide indications related to the functioning of the health parameter monitoring system.

In various implementations, the pharmacological agent may be one of a steroid or an NSAID. The health parameter monitoring system may further comprise a biometric interface that is configured to detect a biometric characteristic of a user of the health parameter monitoring system, and wherein the method may further comprise: detecting a particular biometric characteristic of a user; determining whether the detected particular biometric characteristic is sufficiently similar to a template of the particular biometric characteristic; and allowing the health parameter monitoring system to operate in response to determining that the detected particular biometric characteristic is sufficiently similar to the template of the particular biometric characteristic, or not allowing the health parameter monitoring system to operate in response to determining that the detected particular biometric characteristic is not sufficiently similar to the template of the particular biometric characteristic.

In another general aspect, this document features a health parameter monitoring system comprising: a sensor patch that is configured to be in contact with a human skin surface, wherein the sensor patch comprises a plurality of sensors for measuring physiologic or pathologic parameters; a control unit that is releasably receivable in a cradle of the sensor patch, wherein the control unit is in electrical communication with the plurality of sensors when the control unit is in the cradle, and wherein the control unit and the cradle include physical features that enable a user of the monitoring system to confirm that a particular control unit is designed to be used with a particular cradle; and a cap, wherein the cap is configured to releasably couple with the sensor patch to detain the control unit in the cradle, and wherein the cap includes a user interface that is configured to provide indications related to the functioning of the health parameter monitoring system.

In various implementations, the control unit may comprise a data processing device that is encoded with a computer program, the computer program may comprise instructions that when executed cause (i) data measured by the plurality of sensors to be compared to triggering events that comprise threshold values for health parameters and (ii) an initiation of one or more countermeasures in response to a determination that the data measured by the plurality of sensors meets or exceeds the threshold values of the triggering events. The health parameter monitoring system may further comprise a biometric interface that is configured to detect a biometric characteristic of a prospective user of the health parameter monitoring system.

Particular embodiments of the subject matter described in this document can be implemented to realize one or more of the following advantages. In some embodiments, an integrated health monitoring system based on acquisition of physiologic and pathologic parameters from both non-invasive and implantable sensors can facilitate ambulatory care to promote patient independence and permit a smooth transition from hospital to home care. Through the use of the devices and methods provided herein, in some instances the need for hospital readmissions can be curtailed. In some embodiments, the algorithms provided herein can enable enhanced detection of health issues by using trigger event criteria that are used to initiate corrective actions when the patient's health parameters meet or exceed the trigger event criteria. In particular embodiments, the monitoring system can be configured to reduce the likelihood of improper usage by a patient. In certain embodiments, the patch of the monitoring system can have features that reduce the likelihood for causing skin irritation. Advantageous techniques to ensure a desirable placement of the monitoring device on the patient are also provided. In some embodiments, the algorithms provided herein can be used to increase the accuracy of the data collected by a health monitoring system by detecting and rejecting some signals as artifact noise. In result, the accuracy and effectiveness of health parameter monitoring systems can be improved, overall healthcare costs can be reduced, and patient health and longevity can be enhanced using the devices and methods provided herein.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described herein. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description herein. Other features, objects, and advantages of the invention will be apparent from the description and drawings, and from the claims.

### DESCRIPTION OF THE DRAWINGS

Figures 1A-1C are illustrations of a modular external patient monitoring device in accordance with some embodiments provided herein.
Figure 2 is an illustration of a patient wearing the modular external patient monitoring device of Figures 1A-1C.
Figure 3 is a side cross-sectional view another modular external patient monitoring device in accordance with some embodiments provided herein.
Figures 4A and 4B are illustrations of additional modular external patient monitoring devices in side cross-sectional views in accordance with some embodiments provided herein.
Figures 5A and 5B are illustrations of additional modular external patient monitoring devices in accordance with some embodiments provided herein.
Figure 6 is an illustration of another modular external patient monitoring device in accordance with some embodiments provided herein.

Like reference numbers represent corresponding parts throughout.

### DETAILED DESCRIPTION

This document provides devices and methods for the remote monitoring of patient health parameters. For example, this document provides devices, methods, and algorithms to enhance the operations and efficacy of a remote patient monitoring system. This document also provides devices and methods for monitoring health parameters using a wearable monitoring device that may include non-invasive sensors as well as microsensors that are positioned subdermally in some embodiments. In some embodiments, the wearable health parameter monitoring device includes a disposable component and a reusable component that are electrically and mechanically coupled together to create a functioning monitor device.

The collected health parameter data can be communicated from the monitor device to data collection and analysis systems in a variety of modes. In some implementations, the monitor device can wirelessly transmit data to a cellular telephone that is coupled via a short-range wireless link (e.g., Bluetooth, RF, infrared, etc.) to a transceiver, and the transceiver can communicate over a network such as the internet to a remote monitoring server. In some implementations, a control module from the monitor device can be decoupled from the monitor device and coupled to a base station, computing device, docking device coupled to a computing device, and the like. The health parameter data can then be downloaded from the control module to the base station, and the base station can communicate the data to a remote monitoring server over a network (including, for example, internet, telephone landline or cellular phone networks, Ethernet local area network, etc.). In some embodiments, a combination of such techniques and other techniques known in the art can be used to communicate the health parameter data collected by the monitor device to a remote location for data monitoring and analysis.

In some embodiments, the remote health parameter monitoring system includes a wearable component and a separate computing device that can communicate with each other as well as with a remote monitoring service. In some embodiments, a controller unit in the wearable component performs the personalized algorithms for artifact rejection and for detection of health monitoring events. In other embodiments, the separate computing device performs the personalized algorithms for artifact rejection and for detection of health monitoring events. In some embodiments, both systems can perform such algorithms. While the methods and algorithms provided herein may be described in the context of particular health parameter monitoring systems, it should be understood that the methods, algorithms, and techniques for personalization of health monitoring systems as described in this document can be applied to other monitoring systems or to the data of other monitoring systems.

In one example embodiment, biometric identifiers and associated algorithms are included in remote patient monitoring systems. Such biometric identifiers can personalize a monitor to a particular patient as prescribed by a clinician. Biometric identifiers such as finger print recognition, facial recognition, voice recognition, and other techniques can be used to personalize a health parameter monitor to a particular patient. Such techniques can be used to prevent unintended individuals from using a monitor that is prescribed for use by a particular patient.

In another example embodiment, during set-up and initialization, a monitoring device can acquire biometric data from the individual using the monitor, and the data can be compared to stored data that was acquired from the intended patient. Algorithms can be applied to determine whether the individual using the monitor is likely the intended patient or not. Biometric data including but not limited to ECG, QRS morphology, QRS amplitude, QRS duration, PR interval, impedance, and the like can be used for such comparisons.

As described herein, triggering event criteria can be establish for a patient and programmed in a monitoring device system prescribed for the patient. Such triggering event criteria can be used to determine when the patient is experiencing a health event. That is, when a measured health parameter meets or exceeds the triggering event threshold limit value criteria for that health parameter, the patient is deemed to be experiencing a health event. In such a case, countermeasures can be initiated. Countermeasures can include, but are not limited to, providing a patient alarm, sending an alarm to a remote monitoring service, increasing the monitoring frequency, and alerting emergency medical personnel.

In another example, enhancements for the sensor patch that contacts the patient's skin are provided herein. Some such enhancements can be included to reduce the potential for patient skin irritation. In some cases, pharmacological agents can be administered to the skin from the sensor patch. In another example enhancement to the sensor patch, health parameters, such as glucose, can be monitored transdermally using reverse iontophoresis.

In another example technique, a process for locating the monitoring device at desirable locations on the patient is provided herein. To achieve this, a process of "registration" that involves recording signals from each selected location and programming the monitoring device to recognize the signal quality and pattern of each site as well as the unique signal noise from each site to increase fidelity of recording at each site can be used. Registration can be established by a healthcare professional in an office setting to train the patient on correct placement and use of the device. The registration process can be used to find the anatomical locations with the optimal signal quality to noise ratio, and also to mark those sites for subsequent placement by the patient.

The health parameter data collected from sensors on a patient can be communicated from a wearable monitor device to data collection and analysis systems in a variety of modes. In some implementations, the monitor device can wirelessly transmit data to a cellular telephone that is coupled via a short-range wireless link to a transceiver (e.g., Bluetooth, RF, infrared, etc.), and the transceiver can communicate over a network such as the internet to a remote monitoring server. In some implementations, a control module from the wearable monitor device can be decoupled from the monitor device and coupled to a base station, computing device, docking device coupled to a computing device, and the like. The health parameter data can then be downloaded from the control module to the base station, and the base station can communicate the data to a remote monitoring server over a network (including, for example, internet, Ethernet, telephone landline or cellular phone networks). In some embodiments, a combination of such techniques and other techniques well known in the art can be used to communicate the health parameter data collected by the monitor device to a remote location for data monitoring and analysis.

The sensors used in the monitoring devices provided herein can include a variety of types and configurations. Some sensors are non-invasive. That is, some sensors make contact with the skin surface of the patient. Other sensors penetrate the dermal layers of the patient. Such penetrating sensors may also be referred to herein as "microneedles" or "microsensors." Microneedles can advantageously eliminate signal interference from the patient's skin in some circumstances. Therefore, microneedles may provide enhanced signal reception for parameters including but not limited to electrocardiography (ECG), electroencephalography (EEG), electromyography (EMG), and others.

The monitoring devices provided herein may be used to collect other data types including but not limited to blood pressure, weight, hip waist ratio, oximetry, thoracic, bioimpedance, physical activity, temperature, drug levels, microfluidics (including serum and urine analytes and protein-based assays), respiration rate, heart sounds, voice recordings, heart rate (HR), posture, analyte values such as blood glucose, just to provide a few more examples. Movement or activity may be sensed with appropriate accelerometers or gyroscopes, such as micro electro-mechanical system (MEMS) devices. Such collected data may in turn be synthesized using various algorithms to calculate other health status indicators such as QRS complex values, RR intervals, PVC values, arrhythmia, P wave, and others.

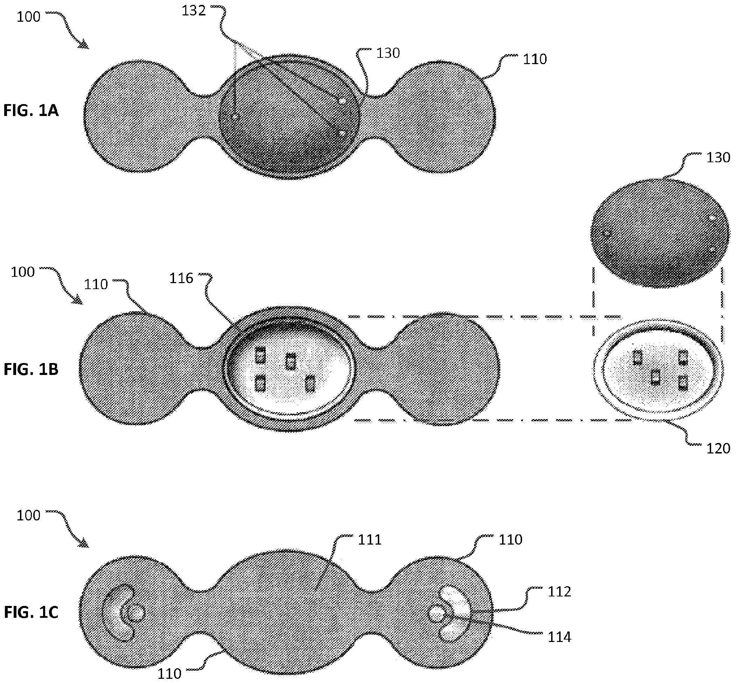

Referring to Figures 1A-1C, a modular external monitoring device 100 is shown in a top view (Figure 1A), an exploded top view (Figure 1B), and a bottom view (Figure 1C). The modular external monitoring device 100 depicted includes a sensor patch 110, control unit 120, and a snap-on monitor 130. Sensor patch 110 includes a central cradle 116 that is a receptacle for releasably receiving control unit 120. With control unit 120 installed in cradle 116, snap-on cap 130 can be installed onto sensor patch 110 over control unit 120 to detain control unit 120 in sensor patch 110 as shown in Figure 1A. Snap-on cap 130 can engage with complementary physical features on the sensor patch 110 so as to snap in place using a mechanical fit, for example. In some embodiments, snap-on cap 130 engages with sensor patch 110 to create a water-resistant seal therebetween.

When control unit 120 is installed in sensor patch 110, electrical connections are made such that control unit 120 is in electrical communication with the sensors that are visible on the bottom of sensor patch 110. Sensor patch 110 includes, in this example embodiment, an ECG electrode 112 and a bioimpedance sensor 114. However, a wide variety of types, configurations and numbers of sensors can be included in sensor patch 110 as described further herein, and as known in the art.

In this embodiment, sensor patch 110 can be a disposable item. Control unit 120 and snap-on cap 130, on the other hand, can be reusable items if so desired. Sensor patch 110 may be used in some circumstances for a period of about a day, or multiple days in other circumstances. When a patient changes sensor patch 110, the patient can discard sensor patch 110 but retain and reuse control unit 120 and snap-on cap 130. This technique of reusing control unit 120 and snap-on cap 130 can provide economic cost savings as compared to discarding the components.

Some portions of modular external monitoring device 100 sensory and monitoring systems are located in sensor patch 110, and other portions are located in control unit 120 and snap-on cap 130. For example, in this embodiment sensor patch 110 includes the sensor devices, such as ECG electrode 112 and bioimpedance sensor 114. A power source such as a battery (not shown), and electrical contacts that mate with complementary contacts on control unit 120 can also be included in the sensor patch 110. Control unit 120 can include microelectronics including but not limited to a CPU, data storage memory, wireless transceiver, power management circuitry, sensor interface circuitry, alarm devices, and complementary contacts that mate with sensor patch 110 and snap-on cap 130. Snap-on cap 130, in addition to contacts that mate with control unit 120, can include user interface devices such as LEDs, a numeric display, a text display, an icon display, audio alarm devices, visual alarm devices, and a combination of such user interface devices.

In some embodiments, the positioning of the electrical contacts of sensor patch 110 and control unit 120 are advantageously configured to ensure mating between sensor patch 110 and control unit 120 in the proper combination as intended. In other words, to prevent an improper combination of a particular control unit 120 with a particular sensor patch 110 that is not designed or intended to function with the particular control unit 120, the electrical contacts can be made to be out of alignment with each other. In that case, the patient will perceive that the improperly assembled monitoring device 100 is not working and take corrective actions to mate a correct control unit 120 with a correct sensor patch 110 as intended. In some embodiments, an improperly mated control unit 120 and sensor patch 110 will provide an alarm notification to the patient. Such an alarm can be an audible tone, a visual indication, a tactile indication (such as vibratory), and a combination of such alarm modes.

This technique of ensuring proper mating between control units 120 and sensor patches 110 can be similarly applied to the mating between control units 120 and snap-on caps 130. That is, the positioning of electrical contacts of control unit 120 and snap-on cap 130 can be configured such that the assembled combination will not function unless a proper snap-on cap 130 is used with a particular control unit 120 as intended.

In some embodiments, other ways of ensuring the proper combination of the components (sensor patch 110, control unit 120, and snap-on cap 130) can be included. For example, color-coding of the components can be used in some cases. In another example, the components can be physically configured to mechanically mate with each other when the proper combinations are made, and their configuration can prevent or hinder the mating of improper combinations of components.

In particular embodiments, ways of ensuring that a particular modular external monitoring device 100 is being used by the intended patient as prescribed and not by other unintended users can be included. In some examples, modular external monitoring device 100 can use biometric identifiers to analyze whether the intended patient is using or attempting to use monitoring device 100. For example, monitoring device 100 can include finger print recognition, voice recognition, retinal scan (via a computer device such as a base station or cellular phone), facial recognition (via a computer device such as a base station or cellular phone), individualized ECG QRS morphology, QRS duration, QRS amplitude, PR interval, and a combination of such and other like factors.

Sensor patch 110 can be made from compliant polymeric materials and can have an adhesive on a bottom surface 111. In some embodiments, sensor patch 110 can comprise a material that is well-suited for the convenient placement on the patient's skin, consistent retention thereon, and non-irritating skin contact. For example, sensor patch 110 can comprise a soft elastomer such as a thermoplastic elastomer, silicone, or the like. Sensor patch 110 can thereby be conveniently soft and/or tacky, flexible, and compliant to assist a user with installation of sensor patch 110. A biocompatible adhesive can be included on portions of bottom surface 111 to cause sensor patch 110 to adhere to the patient's skin, thereby keeping sensors 112 and 114 in proper physical proximity with the patient. Further, the flexibility of sensor patch 110 can help ensure that the sensors 112 and 114 continue to properly function despite most types of physical movements commonly made by the patient.

Snap-on cap 130 can include indicator LEDs 132 (or another type of user interface). LEDs 132 can signal to the patient various messages such as errors, the proper functioning of monitoring device 100, if the monitoring device 100 is transmitting data, and the like. Snap-on cap 130 can be a polymeric material. In some cases, snap-on cap 130 is a more rigid material than sensor patch 110. For example, snap-on cap 130 can be made from any suitable material such as polypropylene, polystyrene, acrylonitrile butadiene styrene (ABS), polycarbonate, PVC, silicone, or the like.

As best seen in FIG. 1B, control unit 120 that includes the memory, CPU, communications, etc. can be reversibly attached/detached to sensor patch 110. Because of this arrangement, a particular control unit 120 can be used with multiple properly configured sensor patches 110, and conversely, multiple properly configured control units 120 can be used with a particular sensor patch 110.

In one example scenario of operating monitoring device 100, a patient can be given two control units 120 that are programmed and personalized identically. The control units 120 are rotated daily. That is, each day the patient removes a control unit 120 and installs the other control unit 120. The following day, the patient repeats the process-again swapping control units 120. In this manner, a particular control unit 120 gets used every other day. This usage of control units 120 can be independent of the patient's frequency of replacing sensor patches 110. In other examples, the frequency of rotation of control units 120 can be other time periods, e.g., about every 12 hours, about every 36 hours, about every two days, about every three days, or for longer periods of time. In some cases, monitoring device 100 can prompt the patient to replace control units 120. In such cases, the prompting can be time-based, event-based, memory storage capacity-based, and so on. Such factors may be programmable into control units 120 by a physician or health technician in keeping with a customized treatment plan for a particular patient.

The control unit 120 that is removed from sensor patch 110 and is not in use on a particular day is installed onto a base station computer system. The base station can be located in the patient's home, at a treatment site, or a combination of such locations. The base station has network access (wired or wirelessly) and a standard AC power supply. In some cases, a cellular phone or other portable computing device can be used instead of or in combination with the base station. The base station then downloads the health parameter data from control unit 120 and either stores the data to the data storage system of the base station or transmits the data to a monitoring service via the network. Further analysis of the data can be performed by the base station, monitoring service, and by health practitioners using the systems. Data can be presented graphically. Trends can be compiled and displayed for analysis. Various types of algorithms can be applied to provide data analysis and diagnostic tools.

In normal use, monitoring device 100 generally records health parameter data on a periodic basis, rather than continuously. In this manner, monitoring device 100 can save battery life, reduce storage requirements, reduce transmission costs, and so on. For example, in some embodiments, health parameter data is routinely recorded during the first about 10 seconds to 1 minute segment at the start of each new hour. The monitoring device 100 can be configured to routinely record health parameter data using any appropriate combination of recording period and on any periodic basis that is desired. In addition, the subsequent analysis of the recorded data may trigger more frequent recording. For example, if the routinely recorded data includes data that meets the definition of a pre-identified trigger event, the frequency of data recordations may be automatically increased in response to meeting the trigger event. In some cases, pre-identified trigger events are defined for a particular patient. In some cases, pre-identified trigger events are based on generally utilized health parameter limits. In some cases, a combination of such types of pre-identified trigger events are used by a single monitoring device 100. When trigger events occur monitoring device 100 can provide an alarm to the patient regarding a possible health event. Such alarms can be audible, visual, tactile (e.g., vibratory), and combinations of such alarm types.

The systems can also allow monitoring device 100 to receive communication. In other words, multi-way communication between control unit 120 and a central data repository (monitoring service, physician/caregivers office, internet sites, etc.) can allow the monitoring service to remotely alter the acquisition frequency, duration, variables being monitored, timing of monitoring (i.e., night versus day versus early morning after waking etc.) of the control unit. The multi-way communication can also allow for alerts, updates, reminders, etc. to be sent to control unit 120 and the patient.

Various configurations of sensors and detectors can be used on monitoring device 100. In some embodiments, thin wire electrodes instead of larger circular electrodes are used. A collection of such wires can be held to the patient's skin via needles or barbs that protrude subdermally. In some cases, the needles can have an angled entry through the patient's skin. In some embodiments, a wicking absorbent membranous covering is included at the patient's skin interface. A microphone can be included on monitoring device 100 for use by the patient to record events, communicate with monitoring personnel, and detect breathing disorders (e.g., apnea). Mechanical stretch sensors (e.g., strain gauges) can be included in monitoring device 100 to assess chest expansions, which can represent respiration rate. One such embodiment can be strip-shaped with each end of the strip affixed to the skin (but a middle portion not affixed to the skin) so that the middle of the strip is stretched by natural chest excursions during respiration. Another monitoring device embodiment can be a band that encircles the chest.

While control unit 120 typically downloads the health parameter data to a base station or equivalent device, in some cases control unit 120, while installed in the sensor patch 110, can send wireless transmissions to the base station or over cellular networks based. These transmissions can be performed based on a determination by control unit 120 that the recorded health parameter data meets or exceeds certain pre-identified triggering events, such as meeting or exceeding a threshold value for a health parameter. Such triggering events can be programmed into control units 120 for the patient. For example, a triggering event may be a particular variability in RR over a short time period, or an ECG QRS morphology, a slow heart rate, a fast heart rate, RR pause, algorithm outcomes, or the like. In some embodiments, a triggering event can be manually created. That is, an individual such as the patient can instigate a recordation period and transmission of health parameter data if the individual so chooses by, for example, activating an event button located on monitoring device 100. One scenario in which this feature may be beneficial, for example, is if the patient feels poorly the patient may choose to record and transmit health parameter data for analysis by a healthcare professional. The event button can be textured for ease of location, and can incorporate features to prevent accidental activation (e.g., the event button can be recessed, covered, etc.). Monitoring device 100 may also respond to voice recognition following an activation of the event button. In some particularly serious scenarios, the patient, healthcare professional, or the monitoring system 100 may request emergency responders (e.g., an ambulance) to attend to the patient as a result of the patient's recorded health parameter data, or in response to the activation of the event button.

Since control unit 120 is removable from sensor patch 110, features can be included to add assurance that a control unit 120 is not mistakenly used with an unintended sensor patch-since the unintended sensor patch would likely not be configured for proper use with control unit 120. These features can also help to prevent a patient's inadvertent use of a monitoring device other than the monitoring device assigned or prescribed for the patient. To accomplish this, in some embodiments control units 120 that are assigned or prescribed for an individual patient can have markings that are a specific color or combination of colors. The corresponding sensor patches 110 can include corresponding color markings. Before use then, the patient can perform an easy visual inspection of the control unit 120 and sensor patch 110 for the color markings that the patient has been assigned. In another example, control units 120 can have particular physical shape or size features and corresponding sensor patches 110 can have complementary physical shape or size features. Using this technique, control units 120 can be mechanically prevented from mating with an improper control unit 120. If the patient attempts to mate the mismatched components, the patient would ascertain the improper mechanical coupling and become aware that the components are not intended for use as an assembled unit.

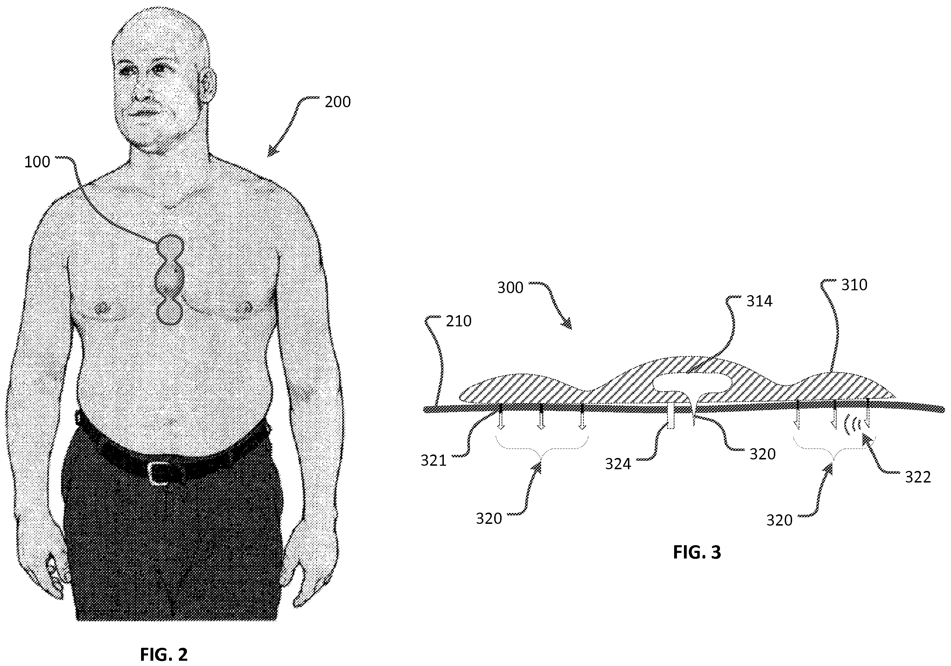

Referring to Figure 2, a patient 200 is illustrated wearing modular external monitoring device 100. Monitoring device 100 is adhered to the skin of patient 200. In this example, monitoring device 100 is on the chest of patient 200 in a position over the sternum to measure heart and respiratory health parameters. This position is less prone to motion artifact than some other locations, because the skeletal and muscle motion above the sternum is generally minimal. Still, in other implementations monitoring device 100 is worn on other areas of patient 200. For example, monitoring device 100 may be worn on the head, abdomen, back, side, extremities, and other suitable locations on patient 200. The location on of monitoring device 100 on patient 200 will depend on the type of health parameter data to be collected.

Various features can be included with monitoring device 100 to enhance its performance and usability. For example, in some embodiments adhesive mixtures or microdepots containing anti-inflammatories (e.g., steroids, NSAIDs, etc.) can be used to prevent local skin irritation during long-term wear. Pharmacological agents may also be administered transdermally using iontophoresis from monitoring device 100. For example, steroids to reduce skin irritation can be delivered using iontophoresis. This technique can be used for automated closed loop drug deliveries. In some embodiments, reverse iontophoresis can be used via monitoring device 100. In some cases, blood glucose monitoring can be performed by reverse iontophoresis via monitoring device 100. Other types of transdermal monitoring can also be performed.

Techniques for promoting beneficial anatomical placement of monitoring device 100 can be used. For example, sites on a patients skin that provide good signal quality can be marked (e.g., using a tattoo - or semi-permanent dye/tattoo, to indicate accelerometer orientation, etc.) so as to facilitate correct placement of subsequent devices by the patient. In some embodiments monitoring device 100 can give an indicator (e.g., lights, sound) when correctly aligned on the patient's anatomy. Multiple sites can be used to allow for rotation of monitoring device 100 placement to reduce the potential for skin irritation.

A process of "registration" can be used that involves recording signals from each selected location and programming the control unit 120 to recognize the signal quality or signal pattern of each site, as well as the unique signal noise from each site to increase fidelity of recording at each site. Registration can be established by a physician or technician in an office setting to train the patient on correct placement and use of monitoring device 100. The registration process can be used to find the anatomical locations with the optimal signal quality to noise ratio, and, in some cases, to mark those sites for subsequent placement by the patient. Communication by control unit 120 with a network connection (e.g., base station, cell phone, etc.) can allow control unit 120 to send signal registration patterns to the monitoring service to verify correct placement of monitoring device 100, and a notification of which placement site is currently being utilized. The monitoring service can send reminders to the patient for rotation of sites to reduce the potential for skin irritation, along with alerts if monitoring device 100 is placed at a site that appears incorrect based on registration patterns.

Temperature or light sensors on monitoring device 100 can detect skin redness and temperature change that may indicate skin irritation and inflammation prompting a change in the position to which monitoring device 100 is adhered. To avoid excessive stress on the monitoring device-such as with women patients that have large breasts-flexible portions can be included in monitoring device 100 to minimize motion of the central part of monitoring device 100. The flexible portions may be an accordion-like portion, an elasticized portion, or other means to mitigate the effects of mechanical forces.

Referring to Figure 3, a cross-sectional side view of another example modular external monitoring device 300 is depicted on the skin 210 of a patient. Monitoring device 300 includes microneedles 320 that can be employed as sensors, injection devices, sampling devices, and for other like purposes. Microneedles 320 can be barbed or otherwise include structures which facilitate adherence to skin 210.

Microneedles 320 penetrate the skin 210 and the distal tips of the mocroneedles 320 reside subdermally. Therefore, microneedles 320, when used as sensors, have enhanced signal reception (e.g., for ECG, EEG, EMG, etc.). The enhanced reception can be due to the elimination of "shielding" by dermal layers to outside interference as well as because of closer proximity to organ to be monitored. In another implementation, microneedles 320 have access to interstitial fluid for sensing electrolytes, glucose, oxygen, pH, temperature, and so on. The portions of microneedles 320 near sensor patch 310 can be insulated portions 321 such that the only electrical recording would come from the exposed electrodes at the distal end of microneedles 320 that are positioned deeper into the tissue. In some cases, this arrangement can reduce signal artifact caused by patient motion or from intermittent contact between skin 210 and a surface electrode (e.g., electrodes 112 and 114 of Figure 1C). Further, there are known electrical potentials that arise from the surface of skin 210 which can be a source of electrical noise. The avoidance of recording from the surface of skin 210 can decrease or eliminate this source of electrical noise.

In some cases, a set of two or more microneedles 320 can function as bipolar electrodes. That is, microneedles 320 can send and receive signals between microneedles 320 as depicted by signal symbol 322, or alternatively between the distal tip and mid-shaft of an individual microneedle 320.

Some embodiments of microneedles 320 can carry fiber optic elements and can transmit light for oximetry sensing. Light attenuation through tissue relates to oxygen tension, using techniques known in the field. The approach of embedding the transmittance and measurement of light within the dermal layers solves the problem of the difficulties with reflective oximetry whereby a patch is applied to a flat region of tissue (e.g. chest or back). In one embodiment, a first microneedle 320 has a side aperture to transmit light. A neighboring microneedle 320 has a complementary side aperture to receive the light transmitted from the first microneedle 320 via the tissue. Thus, the actual technique is transmissive from two neighboring microneedles 320 placed in the tissue. In another embodiment, light is transmitted from a microneedle 320 and the light transmitted through the tissue is received at a sensor on the bottom surface of sensor patch 310. This embodiment could rely on measurement of reflective light or transmissive light, depending on geometric arrangement of the microneedle 320 relative to the base of sensor patch 310.

In some embodiments, microneedles 320 can alternatively be used for drug delivery by injecting medication from a reservoir 314 located within or coupled to sensor patch 310. For example, a drug such as a steroid, lidocain, and others can be beneficially administered to the patient to prevent discomfort and inflammation which could otherwise result from the chronic use of sensor patch 310 and microneedles 320. In another example, an agent can be delivered from reservoir 314 through microneedles 320 to treat a patient's particular detected disorder. Drugs such as quinidine, beta-blocker, amiodarone, insulin, and so on can be used in such applications.

Microneedles 320 may also include accelerometers at distal tips to help with the control of signal noise from the sensors. For example, movement sensed at microneedle 320 tip by an accelerometer can indicate motion and typical signal noise associated with such motion can be anticipated and managed. In some cases, electrical circuitry or software can be used for cancelation, correction, and filtering of the resulting signal to thereby reduce motion artifact. Previous attempts to record signal noise using accelerometers at locations removed from the recording electrode - even by a small amount - have been ineffective due to the lack of correlation between the forces at the accelerometer and at the electrode. An accelerometer in microneedle 320, or at the base of the microneedle 320, can resolve that problem.

In some embodiments, monitor device 300 can also include one or more piezoelectric sensors 324. Piezoelectric sensors 324 can be used to measure bioimpedance which can in turn provide a useful signal for artifact elimination, arrhythmia detection, determination of respiration rate, and other purposes.

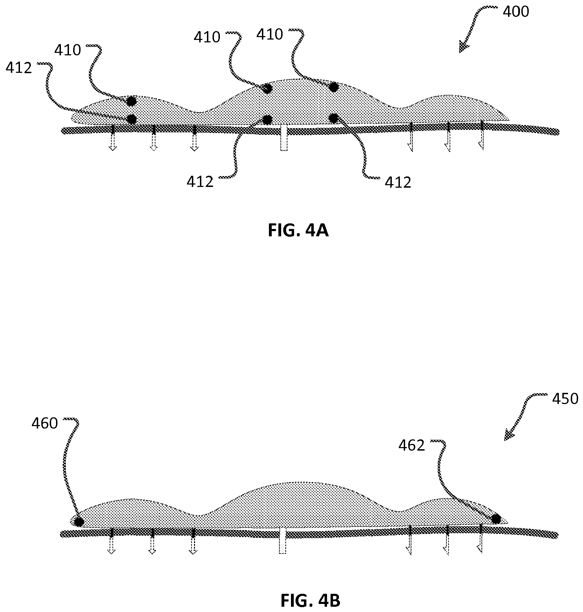

In reference to Figure 4A, a modular external monitoring device 400 is illustrated including one or more upper accelerometers 410 and one or more lower accelerometers 412. In some embodiments, multi-axis gyroscopes can be used in addition to or as a substitute for accelerometers 410 and 412.

Including accelerometers 410 and 412 in monitoring device 400 can provide many advantages. In some embodiments, monitoring device 400 only captures or analyzes data when motion levels as determined by accelerometers 410 and 412 are below certain thresholds levels (so as to avoid motion induced artifact). Accelerometers 410 and 412 can be oriented in multiple arrangements to facilitate several functions (e.g., physiologic monitoring and motion-sensing functions). For example, accelerometers 410 and 412 can be incorporated into the monitoring device 400 platform as independent sensors, or into the electrodes themselves (as described herein). Integration of motion data at the electrode interface may be beneficial when correlated with motion at a distance - away from the electrodes - this would allow for noise subtraction due to motion of monitoring device 400.

Further uses for accelerometers 410 and 412 can include physiologic monitoring. For example, physical activity or inactivity (including "learned" activities) can be measured and correlations of these learned activities with expected changes in other monitored or sensed data inputs can be used to enhance the value of the data collected my monitoring device 400. Signals from accelerometers 410 and 412 can be used to indicate patient falls, long-term inactivity, and levels of activity. Heart sounds and motion permitting event timing and ECG can be detected by accelerometers 410 and 412 in some embodiments. Respiration can be determined based on motion of monitor device 400, bioimpedance changes, or both. Accelerometers 410 and 412 can also be used to determine erect or supine posture. All of these measurements can be combined and cross-checked to determine the presence of artifact and/or increase the sensitivity and specificity of event recording.

Signal noise (artifact) can be very difficult to distinguish from potentially dangerous and rapid heart rhythms. Artifact may be caused by a number of conditions, with two examples being (1) mechanical motion with subsequent myopotentials and (2) poor electrode contact. With poor electrode contact, bioimpedance may be useful particularly when supplemented with data from accelerometers 410 and 412. With physical motion, accelerometers 410 and 412 are useful for determining that artifact is present secondary to motion data from accelerometers 410 and 412. The physical motion that results in ECG artifact can have a characteristic "signature" unique to a specific activity such as walking and other routine activities, tremor, or local skeletal muscle contraction. Thus, rather than performing artifact rejection, the monitoring device 400 would be programmed to detect artifact and classify it as such using the "noise signature" that results from characteristic ECG signals. These unique activity signatures can be taught to the control unit during registration and thereby enhance signal quality and event detection, by artifact detection and rejection.

The location of the accelerometers 410 and 412 can be advantageously selected to enable artifact signal noise detection. Integration of accelerometers onto electrodes as described herein is one good approach. For example, in some embodiments a first accelerometer is embedded in a microneedle or other type of sensor, and a second accelerometer is located in the sensor patch (such as on a circuit board of the control unit). Analysis of the relative motion of these two accelerometers would be useful in developing these characteristic "signatures" of particular types of motion. For example, an individual's stride during walking would likely result in similar motion detected by accelerometers in contact with the skin and by accelerometers on the circuit board, whereas as the type of motion associated with myopotentials (e.g., pectoralis motion) or vibratory motion from riding in a car on a bumpy road may result in different signals from the two accelerometers. If these "noise" motion signals occur in the setting of "high heart rates" it can indicate likely artifact. For example, radial motion such as may be expected with a swinging of the arms or movement of the pectoralis would result in a greater translational motion of the upper accelerometer 410 than a lower accelerometer 412. This difference can be taken advantage of to define a characteristic signature to each type of motion and thus identify specific activities. For example, if a person is traveling in a car and not otherwise moving, then accelerometer 410 would equal accelerometer 412, as both accelerometers 410 and 412 are being equally translated by the car's movement. In contrast, if a person is actively rowing a boat, then accelerometer 410 would be greater than accelerometer 412, thus providing for the detection of this type of activity.

Accelerometer 410 and 412 data in combination with other data such as ECG and impedance data can also be used to indicate poor contact of monitoring device 400 with the patient. In turn, monitoring device 400 can alert the patient or other personnel, reject certain data signals, and so on. The user of multiple accelerometers 410 and 412 can be used to enhance this function. For example, if signals from accelerometers 410 shows motion analogous to accelerometers 412 then skin contact may be lost. Accelerometer 410 motion should be less than that of accelerometer 412 when monitor device 400 is correctly placed and in correct skin contact.

In reference to Figure 4B, a modular external monitoring device 450 is illustrated including one or more first end accelerometers 460 and one or more second end accelerometers 462. In some embodiments, multi-axis gyroscopes can be used in addition to or as a substitute for accelerometers 460 and 462.

Similar to the functionality of accelerometers 410 and 412 as described herein, the relative motion between accelerometers 460 and 462 can indicate monitor device 450 that is being twisted or otherwise configured. Such a motion could indicate a poor contact with the patient's skin such as a detachment of a "wing" or end portion of monitoring device 450.

In some embodiments, a combination of vertically differentiated accelerometers (410 and 412) and horizontally differentiated accelerometers (460 and 462) can be used on a single monitoring device. In addition, other sensors such as a temperature sensor embedded near electrode can be included to enhance detection of improper placement or detachment of monitoring device 450 from the patient's skin. For example, a temperature sensor may indicate a sudden change in temperature or sudden drop in temperature that indicates poor electrode contact with the patient's skin.

Another example technique for personalization of monitoring systems includes a biometric identifier algorithm. This biometric identifier algorithm can be initiated and activated at various times including during set-up, start-up, or restarting of the monitoring system. In one example, the device acquires and stores certain biometric data of the patient to whom the monitoring system is prescribed. Such biometric data can include but is not limited to ECG QRS morphology, QRS amplitude, QRS duration, PR interval, impedance, finger prints, skin markings such as freckles (e.g., using photo recognition), retinal scan, facial recognition, voice recognition, and so on. These parameters provide unique patterns to the individual patient. These biometrics patterns can be used for the purposes of routinely querying the user and verifying that the device is attached to the correct individual and to ensure personalization.

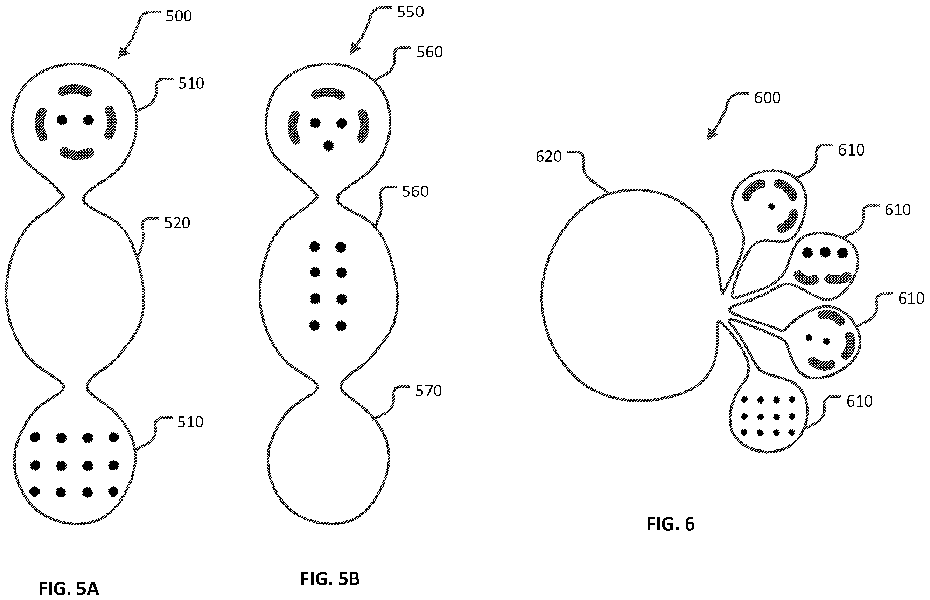

Figures 5A, 5B, and 6 depict additional example configurations of monitoring devices 500, 550, and 600 respectively. As illustrated, a wide variety of configurations are envisioned. The configurations may be tailored to be used on a certain part of a patient's anatomy or on a certain body size. In general, each monitoring device 500, 550, and 600 includes sensor portions 510, 560, and 610 respectively. Further, each monitoring device 500, 550, and 600 includes a location for a control unit 520, 570, and 620 respectively. Monitoring devices 500, 550, and 600 can function as described herein in relation to other embodiments of monitoring devices.

While this specification contains many specific implementation details, these should not be construed as limitations on the scope of any invention or of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described herein as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described herein should not be understood as requiring such separation in all embodiments, and it should be understood that the described program components and systems can generally be integrated together in a single product or packaged into multiple products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In certain implementations, multitasking and parallel processing may be advantageous.

## Claims

1. A method of determining and using a preferred anatomical site at which to locate sensors of a health parameter monitoring system on a patient, the method comprising:
recording health parameter data of the patient from a first anatomical site on the patient to create a first data set;
recording health parameter data of the patient from a second anatomical site on the patient to create a second data set, wherein the first and second anatomical sites are different locations on the patient;
comparing the first data set with the second data set;
determining which of the first data set or the second data set represents a preferred signal;
selecting the first anatomical site as the preferred anatomical site based on determining that the first data set represents the preferred signal or selecting the second anatomical site as the preferred anatomical site based on the determining that the second data set represents the preferred signal;
recording, using the health parameter monitoring system, health parameter data of the patient from an anatomical site that is established by the patient's application of the monitoring system to the patient's anatomy to create a third data set;
comparing the third data set to the data set from the preferred anatomical site; and
based on the comparison of the third data set to the data set from the preferred anatomical site, determining whether the anatomical site that is established by the patient's application of the monitoring system to the patient's anatomy is sufficiently similar to the preferred anatomical site.

2. The method of claim 1, wherein the health parameter monitoring system comprises:
a sensor patch that is configured for being in contact with a skin surface of the patient, wherein the sensor patch comprises a plurality of sensors for measuring physiologic or pathologic parameters of the patient;
a control unit, wherein the control unit is releasably receivable in a cradle of the sensor patch, and wherein the control unit is in electrical communication with the plurality of sensors when the control unit is in the cradle; and
a cap, wherein the cap is configured to releasably couple with the sensor patch to detain the control unit in the cradle, and wherein the cap includes a user interface that is configured to provide indications related to the functioning of the health parameter monitoring system.

3. A method of operating a health parameter monitoring system, the method comprising:
providing the health parameter monitoring system, the health parameter monitoring system comprising:
a sensor patch configured to be in contact with a human skin surface, wherein the sensor patch comprises a plurality of sensors for measuring physiologic or pathologic parameters and a pharmacological agent;
a control unit, wherein the control unit is releasably receivable in a cradle of the sensor patch, and wherein the control unit is in electrical communication with the plurality of sensors when the control unit is in the cradle; and
a cap, wherein the cap is configured to releasably couple with the sensor patch to detain the control unit in the cradle, and wherein the cap includes a user interface that is configured to provide indications related to the functioning of the health parameter monitoring system; and
applying the sensor patch to a skin surface of a patient, wherein the sensor patch administers the pharmacological agent to the patient.

4. The method of claim 3, wherein the pharmacological agent is one of a steroid or an NSAID.

5. The method of claim 3, wherein the health parameter monitoring system further comprises a biometric interface that is configured to detect a biometric characteristic of a user of the health parameter monitoring system, and wherein the method further comprises:
detecting a particular biometric characteristic of a user;
determining whether the detected particular biometric characteristic is sufficiently similar to a template of the particular biometric characteristic; and
allowing the health parameter monitoring system to operate in response to determining that the detected particular biometric characteristic is sufficiently similar to the template of the particular biometric characteristic, or not allowing the health parameter monitoring system to operate in response to determining that the detected particular biometric characteristic is not sufficiently similar to the template of the particular biometric characteristic.

6. A method of claim 3 comprises, a health parameter monitoring system comprising:
a sensor patch that is configured to be in contact with a human skin surface, wherein the sensor patch comprises a plurality of sensors for measuring physiologic or pathologic parameters;
a control unit that is releasably receivable in a cradle of the sensor patch, wherein the control unit is in electrical communication with the plurality of sensors when the control unit is in the cradle, and wherein the control unit and the cradle include physical features that enable a user of the monitoring system to confirm that a particular control unit is designed to be used with a particular cradle; and
a cap, wherein the cap is configured to releasably couple with the sensor patch to detain the control unit in the cradle, and wherein the cap includes a user interface that is configured to provide indications related to the functioning of the health parameter monitoring system.

7. The health parameter monitoring method of claim 6, wherein the control unit comprises a data processing device that is encoded with a computer program, the computer program comprising instructions that when executed cause (i) data measured by the plurality of sensors to be compared to triggering events that comprise threshold values for health parameters and (ii) an initiation of one or more countermeasures in response to a determination that the data measured by the plurality of sensors meets or exceeds the threshold values of the triggering events.

8. The health parameter monitoring method of claim 6, further comprising a biometric interface that is configured to detect a biometric characteristic of a prospective user of the health parameter monitoring system.
